# EUROPÄISCHE PATENTSCHRIFT

(11) **EP 1 248 588 B1**
(45) Veröffentlichungstag und Bekanntmachung des Hinweises auf die Patenterteilung: **11.05.2005**
(21) Anmeldenummer: 01900446.4
(22) Anmeldetag: 15.01.2001
(51) Int. Cl.: A61K 6/10, A61K 6/087

(54) **ZUBEREITUNGEN AUF POLYETHERBASIS UND DEREN VERWENDUNG**
POLYETHER BASED PREPARATION AND THE USE THEREOF
PREPARATIONS A BASE DE POLYETHER ET LEUR UTILISATION

(30) Priorität: 17.01.2000 DE 10001747
(43) Veröffentlichungstag der Anmeldung: 16.10.2002
(73) Patentinhaber: 3M ESPE AG, 82229 Seefeld (DE)
(72) Erfinder: WANEK, Erich, 86916 Kaufering (DE); ECKHARDT, Gunther, 82346 Frieding (DE); ROAS, Peter, 82396 Pähl/Fischen (DE)
(86) Internationale Anmeldenummer: PCT/EP2001/000395
(87) Internationale Veröffentlichungsnummer: WO 2001/052792

(56) Entgegenhaltungen:
- EP-A- 0 421 371
- DE-A- 3 246 654
- DE-A- 19 711 514
- DE-A- 19 740 234
- DE-A- 19 942 459

## Beschreibung

Die Erfindung betrifft Zubereitungen auf der Basis von Aziridinopolyethem und ihre Verwendung zur Herstellung von Dentalmaterialien, insbesondere von Abformmaterialien.

Die Herstellung von Polyetherderivaten und ihre Verwendung in Dentalmaterialien ist seit langem bekannt. So beschreibt beispielsweise die DE-C-174 58 10 die Herstellung von Formkörpem auf der Basis von Aziridinopolyethern.

In den Schriften DE-C-32 46 654, EP-A-0 421 371 und EP-A-0 110 429 ist die Verwendung von Aziridinopolyethern in Polyetherabformmassen beschrieben.

Die Abformung der konkreten Verhältnisse im Mund des Patienten mit Hilfe geeigneter Abformmassen ist die Voraussetzung zur Herstellung von passgenauen Prothesen, Kronen und Brücken, Inlays und Onlays.

Von den bekannten Abformmassen zeichnen sich die auf Aziridinopolyethern basierenden Massen durch ihren hydrophilen Charakter aus, was eine sehr hohe Präzision der Abdrücke möglich macht.

Nachteilig ist an diesen Massen allerdings, dass sie sich nicht allzu leicht entformen lassen. Das heißt, dass die Entformbarkeit des Abdrucks bei der Abdrucknahme sowie die Entformbarkeit des Gipsmodells nach dem Ausgießen des Abdrucks nicht zufriedenstellend sind.

In der DE-A-197 40 234 werden Dentalmassen auf der Basis von Polyetherderivaten beschrieben, die dadurch gekennzeichnet sind, dass ihr Gehalt, an cyclischen oligomeren Polyethem kleiner als 5,0 % ist. Es wird beschrieben, dass die in den Polyethermassen vorhandenen cyclischen Polyetheroligomeren für die schlechte Entformbarkeit des Abdrucks bei der Abdrucknahme sowie eine schlechte Entformbarkeit des Gipsmodells nach dem Ausgießen des Abdrucks verantwortlich sind.

Aufgabe der vorliegenden Erfindung ist es, Dentalmassen auf der Basis von Aziridinopolyethern bereitzustellen, die sich durch eine erleichterte Entformbarkeit auszeichnen.

Diese Aufgabe wird gelöst durch Zubereitungen und daraus hergestellten Dentalmassen, wie sie in den Ansprüchen beschrieben sind.

Es wurde gefunden, dass man die Entnehmbarkeit des Abdrucks und die Entformbarkeit des Gipsmodells unter Beibehaltung der hohen Abdruckpräzision auch bei Aziridinopolyether-basierten Abformmaterialien mit einem verringerten Gehalt von cyclischen Polyethern noch weiter verbessern kann, wenn man durch die Auswahl der Wirkstoffe und der Konzentrationsverhältnisse der Wirkstoffgruppen in der aus der Katalysatorkomponente und der Basiskomponente angemischten Abformungszubereitung niedrige Härten der abgebundenen Elastomermasse einstellt.

Dabei war überraschend und nicht vorhersehbar, dass deutlich niedrigere Härtewerte bei etwa gleicher Konsistenz der angemischten Abformzubereitungen im Vergleich zu den bisher beschriebenen Abformmassen auf Basis von Aziridinopolyethern erreichbar sind und dass sich das Anfließverhalten der erfindungsgemäßen Abformzubereitungen gegenüber den Abformmassen entsprechend dem Stand der Technik deutlich verbessert, was sich in einer höheren Zeichnungsschärfe auswirkt.

Damit werden auch schwierige klinische Situationen, wie die Abformung. subgingival gelegener Präparationsgrenzen in Anwesenheit von Blut und Speichel, besser handhabbar.

Dentalmassen auf Polyetherbasis im Sinne dieser Erfindung haben vorzugsweise Shore A-Härten im Bereich von 45 bis 55.

Die aus den erfindungsgemäßen Zubereitungen formulierten Dentalmassen umfassen insbesondere zwei Komponenten, nämlich die Katalysatorkomponente und die Basiskomponente.

Dabei enthält die Katalysatorkomponente mindestens eine Startersubstanz und die Basiskomponente die Aziridinopolyether.

Die einzelnen Wirkstoffe oder Wirkstoffgruppen, die zur Erzielung einer guten Verarbeitbarkeit und zur Erlangung der gewünschten Eigenschaftskombination der ausgehärteten Elastomeren geeignet sind, können anteilig in der Katalysatorkomponente und der Basiskomponente oder in nur einer der Komponenten enthalten sein.

Die Aufteilung dieser Wirkstoffe auf die Komponenten richtet sich nach dem angestrebten Mischungsverhältnis, der leichten Anmischbarkeit und der ausreichenden Lagerbeständigkeit der so entstehenden Substanzgemische in den getrennt gelagerten Komponenten.

Diese Aufteilung kann durch entsprechende Versuchsreihen optimiert werden.

Die erreichbaren Eigenschaften während der Abdrucknahme und in den ausgehärteten Abformmaterialien hängen überwiegend von der Mischgüte und der Gesamtzusammensetzung der Abformzubereitung ab.

Eine ausreichende Mischgüte wird bei der Handanmischung beispielsweise durch, intensives Anspateln auf einem Anmischblock bis zu einer gleichmäßigen Färbung des Gemisches aus den unterschiedlich eingefärbten Komponenten erreicht.

Vorteilhaft ist die praktische Durchführung der Mischung mittels kontinuierlicher Mischer, meist bestehend aus einer Volumendosiereinheit und statischen oder dynamischen Mischelementen.

Mit diesen Geräten ist eine ausreichende Mischgüte erreichbar, was leicht an der gleichmäßigen Färbung kontrolliert werden kann.

Üblicherweise wird bei Dentalmassen auf Polyetherbasis das Volumenmischverhältnis zwischen Katalysatorkomponente und Basiskomponente auf Werte von 1 : 1 bis 1 : 10 eingestellt, wobei die Einstellungen 1 : 2 und 1 : 5 besonders bevorzugt sind.

Die erfindungsgemäßen Dentalmassen auf Polyetherbasis mit verbesserter Entformbarkeit und verbessertem Anfließverhalten werden aus Zubereitungen erhalten, enthaltend:
(A) 30 bis 56 Gew.-%, bevorzugt 41 bis 54 Gew.-% von Aziridinopolyethern mit einem Gehalt von cyclischen Polyethern kleiner als 5,0 Gew.-% und bevorzugt kleiner als 0,9 Gew.-%;
(B) 30 bis 45 Gew.-%, bevorzugt 30 bis 42 Gew.-% von Verbindungen, die eine Weichstellung der ausgehärteten Dentalmassen bewirken;
(C) 10 bis 15 Gew.-%, bevorzugt 12 bis 14 Gew.-% Füllstoffe;
(D) 4 bis 10 Gew.-%, bevorzugt 4 bis 7 Gew.-% an weiteren Wirkstoffen, wie Farbmitteln, Aromen, Startern, Verzögerem, Beschleunigern und Tensiden;
mit den Maßgaben, dass
- das Gewichtsverhältnis zwischen den Bestandteilen (A) und (C) zu den Verbindungen des Bestandteils (B) 1,2 bis 2,1, bevorzugt 1,3 bis 1,9 beträgt,
- der Bestandteil (B) aus Verbindungen mit Molmassen kleiner 500 g/Mol (B1) und aus Trisacylestern des Glycerins nicht tierischen Ursprungs mit Molmassen zwischen 500 bis 2000 g/Mol (B2) sowie aus Verbindungen mit Molmassen größer 2000 g/Mol (B3) besteht und
- das Gewichtsverhältnis zwischen (B1) und (B3) 1 : 0,8 bis 1 : 2,3 beträgt.

Die gemäß Bestandteil (A) eingesetzten Aziridinopolyethem können aus Polyetherpolyolen hergestellt werden, die bevorzugt durch Copolymerisation von Tetrahydrofuran und Ethylenoxid im Molverhältnis 10 : 1 bis 1 : 1, vorzugsweise 5 : 1 bis 3 : 1 in Gegenwart starker Säure, wie beispielsweise Borfluorid-Etheraten hergestellt werden.

Es ist ebenfalls möglich, Polyetherpolyole einzusetzen, die neben Tetrahydrofuran-Einheiten auch Ethylenoxid-Einheiten und bzw. oder Propylenoxid-Einheiten enthalten.

Die Polyetherpolyole besitzen mindestens 2 Hydroxylgruppen, können aber auch bis zu 20 Hydroxylgruppen pro Molekül enthalten.

Die Molmassen (Mₙ) der zur Funktionalisierung eingesetzten Polyetherpolyole liegen üblicherweise im Bereich von 500 bis 20000 g/Mol, bevorzugt im Bereich von 2000 bis 10000 g/Mol. Die Funktionalisierung mit Aziridinogruppen kann beispielsweise nach dem in der DE-C-1 745 810 beschriebenen Verfahren erfolgen.

Zur Herstellung der erfindungsgemäßen weichen Dentalmassen werden als Bestandteil (A) bevorzugt Bis-Aziridinopolyether mit Aziridinoäquivalentmassen von 2000 bis 4000 g/Äquivalent verwendet, wobei der Polyetherteil aus Oxytetramethylen- und Oxydimethylen-Einheiten vorzugsweise im Verhältnis 4 : 1 bis 3 : 1 besteht und der Anteil an oligomeren cyclischen Ethem in den Bis-Aziridinopolyethern kleiner als 0,5 Gew.-%, bevorzugt kleiner als 0,3 Gew.-% ist.

Die Entfernung der cyclischen oligomeren Polyether kann sowohl auf der Verfahrensstufe der Polyetherpolyole als auch nach deren Funktionalisierung mit Aziridinogruppen erfolgen, wobei destillative und extraktive Verfahren oder die Membrantrennung anwendbar sind.

Ein typisches Verfahren zur Herstellung eines Aziridinopolyethers, der weitgehend von oligomeren cyclischen Ethem befreit wurde, ist im Herstellungsbeispiel 2 von DE-A-197 40 234 beschrieben.

Zur Erzielung der gewünschten Eigenschaften enthalten die Abformmassen 30 bis 45 Gew.-% an Verbindungen, die eine Weichstellung der ausgehärteten Dentalmassen bewirken.

Solche Verbindungen können sowohl typische Weichmacher sein, wie sie auch für andere polymere Systeme angeboten werden, wie Ester von mehrwertigen Carbonsäuren, polyaromatische Verbindungen und Sulfonsäureester oder Verbindungen, die außer der Weichstellung auch andere Effekte wie beispielsweise Tensidwirkung, Erhöhung der Standfestigkeit und Verbesserung des Fließverhaltens bewirken.

Es wurde nun überraschenderweise gefunden, dass das gewünschte Eigenschaftsbild weicher Dentalmassen auf Polyetherbasis, ausgehend von im Gehalt an oligomeren cyclischen Polyethern stark reduzierten Aziridinopolyethern insbesondere dann erreicht werden kann, wenn spezielle Verbindungsklassen dieser die Weichstellung bewirkenden Verbindungen eingesetzt werden und ein bestimmtes Verhältnis dieser Verbindungsklassen untereinander eingehalten wird.

So enthalten die erfindungsgemäßen Zubereitungen gemäß Bestandteil (B) drei Verbindungsklassen von Verbindungen, die eine Weichstellung der ausgehärteten Dentalmassen bewirken, nämlich
(B1) typische Weichmacher mit Molmassen kleiner 500 g/Mol,
(B2) bei Raumtemperatur feste Trisacylglyceride mit Molmassen im Bereich von 500 bis 2000 g/Mol,
(B3) bei Raumtemperatur flüssige Polymere mit Molmassen über 2000 g/Mol,
wobei das Gewichtsverhältnis zwischen (B1) und (B3) 1 : 0,8 bis 1 : 2,3 beträgt. Als Verbindungen gemäß dem Anteil (B1) werden unterschiedliche Weichmachertypen eingesetzt, darunter typische Weichmacher vom Estertyp, wie:
- C₁₂- bis C₁₅-Alkyllactate,
- Ethyl- oder Butylester der Citronensäure oder der Acetylcitronensäure,
- Phthalsäureester längerer verzweigter Alkohole, wie Bis(2-ethylhexyl)-phthalat oder Phthalsäurepolyester,
- C₂- bis C₁₈-Dialkylester von C₂- bis C₆-Dicarbonsäuren, wie Bis(2-ethylhexyl)-adipat, Dioctylmalat, Diisopropyladipat,
- aromatische und aliphatische Sulfonsäureester, wie C₂- bis C₂₀-Alkylsulfonsäureester des Phenols oder von C₁- bis C₁₈-Alkanolen
und typische aromatische Weichmacher, wie:
- Polyphenyle in einem weiten Viskositätsbereich, einschließlich wachsartiger Polyphenyle (Fa. Monsanto),
- Dibenzyltoluol,
- Isomerengemische von C₂₀- bis C₃₀-Aromaten,
wobei die Verwendung von Gemischen aus Weichmachern des Estertyps und des aromatischen Typs bevorzugt ist.

Ein Beispiel für ein bevorzugtes Gemisch ist Acetyltributylcitrat und Dibenzyltoluol.

Gemäß Bestandteil (B2) werden Trisacylester des Glycerins nicht tierischen Ursprungs eingesetzt.

Der Bestandteil (B2) kann aus modifizierten Fetten pflanzlichen Ursprungs, wie beispielsweise aus hydriertem Palmöl oder Sojaöl oder aus synthetischen Fetten bestehen.

Geeignete Fette sind in der DE-A-197 11 514 beschrieben, auf die hier vollinhaltlich Bezug genommen wird. Besonders geeignet sind Avocadoöl, Baumwollsaatöl, Erdnussöl, Kakaobutter, Kürbiskemöl, Leinöl, Maiskeimöl, Olivenöl, Palmöl, Reisöl, Rüböle, Saffloröl, Sesamöl, Sojaöl, Sonnenblumenöl, Traubenkemöl, Weizenkeimöl, Bomeotalg, Fulwatalg, Hanföl, Illipébutter, Lupinienöle, Kandelnussöl, Kapoköl, Katiaufett, Kenafsamenöl, Kekunaöl, Mohnöl, Mowrahbutter, Okraöl, Perillaol, Salbutter, Sheabutter und Tungöl, sofern diese Fette vor ihrer Verwendung gehärtet wurden. Als geeignet gehärtete Fette werden solche betrachtet, deren Jod-Zahl (gemessen nach der Norm DGF C-V 11b) kleiner als 20 ist. Besonders bevorzugt sind Fette, deren Jod-Zahl kleiner als 5 ist. Die Durchführung von Fetthärtungen ist beispielsweise in "Ullmanns Enzyklopädie der industriellen Chemie", 4. Aufl., Band 11, S. 469 beschrieben. Ebenso verwendbar sind Mischungen dieser natürlich vorkommenden Fette, sowie künstlich hergestellte Fette, wie Softisan 154 oder Dynasan 118 (Fa. Hüls). Die Herstellung derartiger künstlicher Triacylglyceride ist für den Fachmann relativ einfach und kann beispielsweise aus Glycerin und den entsprechenden Fettsäuremethylestern erfolgen. Derartige Veresterungsreaktionen sind u. a. in "Houben-Weyl, Methoden der Organischen Chemie", Bd. E5/Teil 1, S. 659 ff. beschrieben. Bevorzugte Triacylglyceride entsprechen der Formel:

R²-O-CH₂-CH(OR¹)-CH₂-O-R³

in welcher R¹, R² und R³ unabhängig voneinander C₁₁H₂₃CO, C₁₃H₂₇CO, C₁₅H₃₁CO oder C₁₇H₃₅CO bedeuten. Auch Gemische solcher Triacylglyceride kommen in Betracht.

Gemäß Bestandteil (B2) der vorliegenden Erfindung werden bevorzugt künstliche Fette mit einem Steaorylgehalt von mehr als 65 Gew.-% des Triglycerids eingesetzt.

Eine besondere Wirkung geht von den flüssigen polymeren Verbindungen in den erfindungsgemäßen Zubereitungen aus. Diese Verbindungen mit Molmassen über 2000 g/Mol können unterschiedlichen Verbindungstypen, wie dem Polyethertyp, Polyestertyp, Polyurethantyp, Polycarbonattyp, Polyolefintyp angehören, wobei als Endgruppen Hydroxyl-, Ether-, Alkyl- und Acylgruppen bevorzugt sind.

Die Auswahl der Endgruppen und ggf. weiterer funktioneller Gruppen erfolgt vorzugsweise derart, dass während der Lagerung der beiden Komponenten und nach der Mischung keine unerwünschten Reaktionen ablaufen.

Besonders bevorzugte Endgruppen sind die primäre und die sekundäre OH-Gruppe sowie die Acetylgruppe.

Eine spezielle Verbindungsklasse von flüssigen Polymeren stellen solche vom Polyethertyp dar.

Hierbei zeichnen sich solche Polyether besonders aus, die die gleiche oder eine ähnliche Molmasse besitzen, wie die in Bestandteil (A) verwendeten Aziridinopolyether.

Bis-Hydroxyl- oder Bis-Acetyl-polyether aus Oxytetramethylen- und Oxydimethylen-Einheiten im Verhältnis 4 : 1 bis 3 : 1 und Molmassen im Bereich von 3000 bis 8000 g/Mol und einem Anteil an oligomeren cyclischen Ethem kleiner als 0,5 Gew.-% sind besonders bevorzugt.

Im Gemisch mit diesen speziellen Polyethem oder auch als alleinige Verbindungen gemäß Bestandteil (B3) können auch Polypropylenoxidpolyole und/oder Copolymerisate und/oder Blockcopolymerisate von Ethylenoxid und Propylenoxid mit Hydroxyl- oder Acetyl-Endgruppen eingesetzt werden.

Bei den Blockcopolymerisaten mit Molmassen größer 2000 g/Mol kann zusätzlich die lösungsvermittelnde Wirkung dieser tensidartigen Verbindungen genutzt werden.

Weiterhin kann durch die Wahl und die Mischung der vorgenannten Polyetherderivate das Fließverhalten und die notwendige Einstellung von Hydrophilie und Hydrophobie der gemischten Zubereitungen entscheidend beeinflusst werden.

Die erfindungsgemäßen Zubereitungen enthalten als Bestandteil (C) 10 bis 15 Gew.-% an verstärkend wirkenden Füllstoffen.

Für diesen Zweck können organische und anorganische Feststoffe eingesetzt werden, die in den Substanzgemischen der jeweiligen Komponente während der notwendigen Lagerung keine unerwünschten Reaktionen hervorrufen und nach der Mischung der getrennt gelagerten Komponenten den Abbindeverlauf nicht beeinträchtigen.

Besonders bewährt haben sich Füllstoffe mit einem SiO₂-Anteil über 90 Gew.-%, wie Quarzmehl und feinteilige Kieselsäuren synthetischen oder natürlichen Ursprungs.

Bevorzugt sind pyrogene Kieselsäuren und Fällungskieselsäuren, die meist in oberflächenmodifizierter Form eingesetzt werden sowie Diatomeenerde unterschiedlicher Fundstellen.

Gemische von aufbereiteter Diatomeenerde mit einem pH-Wert der 5 %-igen wässrigen Suspension von 8 bis 10 und pyrogener, oberflächenmodifizierter Kieselsäure mit BET-Oberflächen von 100 bis 300 m²/g sind besonders bevorzugte Füllstoffe gemäß Bestandteil (C).

Weiterhin enthalten die erfindungsgemäßen Zubereitungen gemäß Bestandteil (D). 4 bis 10 Gew.-% an weiteren Wirkstoffen wie Farbstoffen und Farbpigmenten, an Aromen und Geschmackskorrigentien, an Startersubstanzen wie beispielsweise Sulfoniumsalzen oder Säuren, an aminischen oder alkalischen Verzögerem, an beschleunigend wirkenden Verbindungen und an weiteren nicht polymeren Tensiden.

Der Einsatz der erfindungsgemäßen Zubereitungen kann in sehr unterschiedlichen zahnmedizinisch oder zahntechnisch verwendeten Dentalmassen erfolgen. Bevorzugte Einsatzgebiete solcher Dentalmassen sind die einphasige und die zweiphasige zahnmedizinische Abformung und die Bissregistrierung.

Gegenstand der Erfindung sind auch Behältnisse und Mischvorrichtungen, enthaltend aus den erfindungemäßen Zubereitungen hergestellte Massen, insbesondere Dentalmassen, wie Kartuschen, Beutel, Abformlöffel, statische und dynamische Mischer bzw. Mischgeräte.

Die Erfindung wird anhand der folgenden Beispiele weiter erläutert, ohne sie auf diese zu beschränken.

### Beispiele

### Herstellungsbeispiele Basis- und Katalysatorpasten

Die Herstellung eines Bis-Aziridinopolyethers mit einem niedrigen Gehalt an cyclischen oligomeren Polyethem, ausgehend von einem Bis-Aziridinopolyether, der gemäß der DE-C-174 58 10 erhalten wurde, erfolgt gemäß Herstellungsbeispiel 2 von DE-A-197 40 234.

Die Bestimmung des Restgehalts der cyclischen oligomeren Polyether in diesen Aziridinopolyether wird gaschromatographisch nach der in DE-A-197 40 234 beschriebenen Methode vorgenommen.

Aus diesem Bis-Aziridinopolyether mit einer zahlenmittleren Molmasse von 6100 g/Mol und einem Einbauverhältnis von Ethylenoxid- zu Tetrahydrofuran-Einheiten von 1 : 3,6 sowie einem Restgehalt von 0,25 Gew.-% an oligomeren cyclischen Polyethem werden die in Tabelle 1 charakterisierten Basiskomponenten auf einem Laborkneter im 500 g-Maßstab hergestellt.

Die Herstellung der in Tabelle 2 charakterisierten Katalysatorpasten erfolgt im Laborkneter im 100 g-Maßstab.

Zur Ermittlung der in den Tabellen 4 und 5 angegebenen Eigenschaften werden die Komponenten gemäß den Beispielen in Tabelle 3 auf dem Block gemischt.

### Herstellung von Abdrücken

Die Katalysatorkomponenten und die Basiskomponenten wurden im angegebenen Verhältnis auf dem Anmischblock gemischt, die Mischungen auf ein Metalltray übertragen und der gefüllte Abdrucklöffel in den Mund des Probanden eingeführt.

In den erfindungsgemäßen Beispielen 1, 2, 3, 5, 6, 7 und in den Vergleichsbeispielen 1 und 2 wurde der Abdrucklöffel ausschließlich mit der angemischten Masse befüllt (Monophasentechnik).

Im erfindungsgemäßen Beispiel 4 wurde der Abdrucklöffel mit der Masse des erfindungsgemäßen Beispiels 3 befüllt, die Zähne des Probanden hingegen mit der Masse des erfindungsgemäßen Beispiels 4 umspritzt (Doppelmischtechnik).

Im Vergleichsbeispiel 3 wurde der Abdrucklöffel mit der Masse des Vergleichsbeispiels 1 befüllt, die Zähne des Probanden hingegen mit der Masse des Vergleichsbeispiels 3 umspritzt (Doppelmischtechnik).

Nach einer Abbindezeit von 6 Minuten, gerechnet von Mischbeginn, wurden die Abdrücke entnommen.

Die Entnehmbarkeit des Abdrucks aus dem Mund des Probanden wurde gemäß der in DE-A-197 40 234 beschriebenen Vorgehensweise jeweils an acht Probanden mit unterschiedlichen Gebisssituationen von zwei Durchführenden bewertet und die subjektiven Eindrücke gemittelt.

Es wurde folgendes Bewertungsschema für die Entnehmbarkeit aus dem Mund zu Grunde gelegt:

1 (sehr gut), 2 (gut), 3 (genügend), 4 (mangelhaft), 5 (schlecht).

Im Anschluss an die Bewertung der Entnehmbarkeit wurde im Abdruck das Anfließverhalten der angemischten Abformzubereitung durch Betrachtung der Sulcuswiedergabe einschließlich Präparationsgrenze und Beurteilung der Details der Oberflächenstruktur bewertet.

| **Bewertung** | Kriterien |
|---|---|
| 1 | Sulcus und Präparationsgrenze werden fein auslaufend absolut fehlerfrei wiedergegeben. Perfekte Wiedergabe der Oberflächendetails der Zahnhartsubstanz (Präparationsriefen, Randspalt bei Füllungen, Übergänge). Schleimhautprofil ist exakt dargestellt. |
| 2 | Sulcus und Präparationsgrenze werden gut wiedergegeben. Gute Wiedergabe der Oberflächendetails der Zahnhartsubstanz (Präparationsriefen nicht vollständig aufgelöst, Randspalte gut feststellbar). |
| 3 | Sulcus und Präparationsgrenze sind nicht durchgehend wiedergegeben. |

### Bestimmung der Entformbarkeit des Abdrucks vom Gipsmodell

Die Bestimmung der Entformbarkeit wurde unter Verwendung eines speziell präparierten Unterkiefer-Kunststoff-Modells durchgeführt.

Bei diesem Unterkiefer-Kunststoff-Modell wurden die 6 Frontzähne (zahnärztliche Bezeichnung = 43, 42, 41, 31, 32, 33) so präpariert, dass ein sehr starker Substanzverlust bei den präparierten Zähnen 41 und 31 gegeben war.

Weiterhin wurde der Prämolar 45 mit starken Unterschnitten versehen und als Beispiel eines alleinstehenden, durch Paradontalschädigung stark substanzreduzierten Zahnes in die Bewertung einbezogen.

Das Kunststoff-Modell wurde mit den angegebenen Abformzubereitungen abgeformt und die so erhaltenen Abdrücke wurden nach einer Liegezeit von einer Stunde mit einem Stone-Gips ausgegossen.

Nach einer Gips-Aushärtezeit von 24 Stunden wurde der Abdruck vom Modell entformt und bewertet.

| Bewertung | Kriterien |
|---|---|
| 1 | Das Gipsmodell konnte schnell und ohne großen Kraftaufwand entformt werden und wies keine Beschädigungen (Brüche) auf. |
| 2 | Das Gipsmodell wies keine Beschädigungen auf. |
| 3 | Nur die beiden sehr stark substanzreduzierten und deshalb sehr bruchgefährdeten Zähne (41 und 31) werden bei der Entformung beschädigt (gebrochen). |
| 4 | Mehr als drei der präparierten Zähne (43, 42, 41, 31, 32, 33) werden bei der Entformung beschädigt (Bruchschädigung). |
| 5 | Die präparierten Zähne (43, 42, 41, 31, 32, 33) werden bei der Entformung beschädigt (Bruchschädigung) und der alleinstehende Prämolar (45) reißt vom Gipsmodell ab. |

Die Bestimmung der Entformbarkeit wurde jeweils von drei Personen vorgenommen. Die drei Einzelwerte wurden gemittelt.

Die Zubereitungen der Erfindungsbeispiele bezüglich der aushärtenden Abformzubereitungen entsprechen in allen Punkten den erfindungsgemäßen Kriterien und Maßgaben, während die Vergleichsbeispiele diese Kriterien und Maßgaben in mindestens einem Punkt nicht erfüllen.

Die Shore A-Härte-Werte der Erfindungsbeispiele liegen im gewünschten Bereich für weiche Abformmassen (Shore A: 45 bis 55, gemessen nach DIN 53505).

Die Vorteile der Entnehmbarkeit aus dem Mund und Entformbarkeit des Gips-Modells, die mit den Zubereitungen der erfindungsgemäßen Beispiele 1 bis 7 erreicht werden, gehen aus Tabelle 4 hervor.

Die Vorteile im Anfließverhalten der angemischten Abformzubereitungen (Zeichnungsschärfe) gehen aus Tabelle 5 hervor.

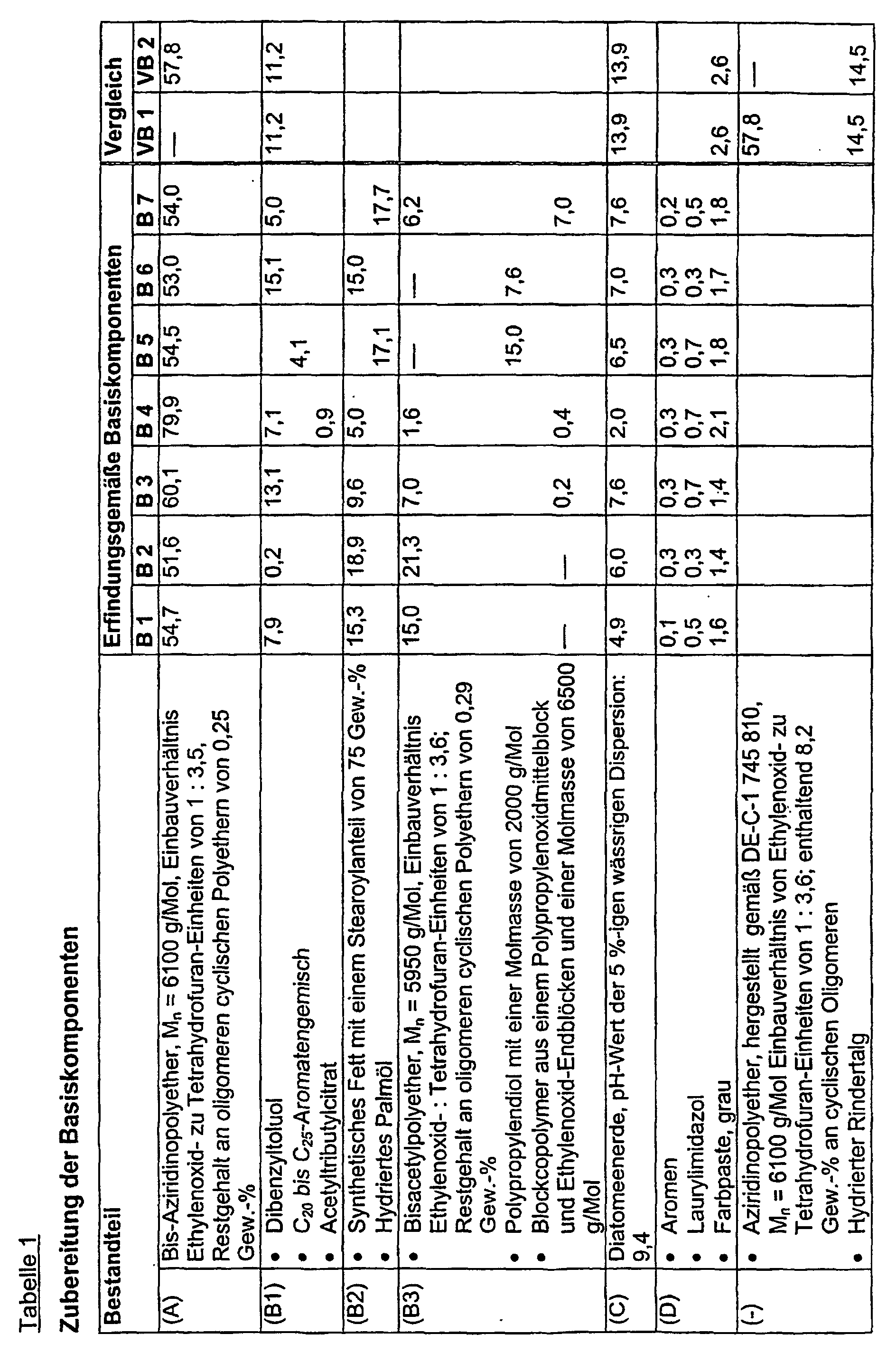

**Tabelle 2**

| **Zubereitung der Katalysatorkomponenten** | | | | |
|---|---|---|---|---|
| **Bestandteil** | | **Erfindungsgemäße Katalysatorkomponenten** | | **Vergleich** |
| | | **K1** | **K2** | **VK 1** |
| (B1) | Acetyltributylcitrat | 39,9 | 18,9 | 32,0 |
| | Dibenzyltoluol | | 5,5 | |
| (B2) | Synthetisches Fett mit einem Steaorylanteil von 75 Gew.-% | ― | 2,0 | |
| (B3) | • Bisacetylpolyether, Mₙ = 5950; Einbauverhältnis: Ethylenoxid- : Tetrahydrofuran-Einheiten von 1 : 3,6; Restgehalt an oligomeren cyclischen Polyethem von 0,29 Gew.-% | 0,5 | 28,9 | |
| | • Blockcopolymer aus einem Polypropylenoxidmittelblock und Polyethylenoxid-Endblocken und einer Molmasse von 6500 g/Mol | 3,5 | 2,7 | 5,8 |
| (C) | • Diatomeenerde | 12,1 | 23,0 | 9,5 |
| | • Pyrogene Kieselsäure, hydrophob modifiziert, BET-Oberfläche 160 m² / g | 24,1 | 5,3 | 19,1 |
| (D) | • Sulfoniumstarter gemäß Beispiel 27 der DE-A-2 515 593 | 19,3 | 13,4 | 32,9 |
| | • Farbpaste, rot | 0,6 | 0,3 | 0,7 |

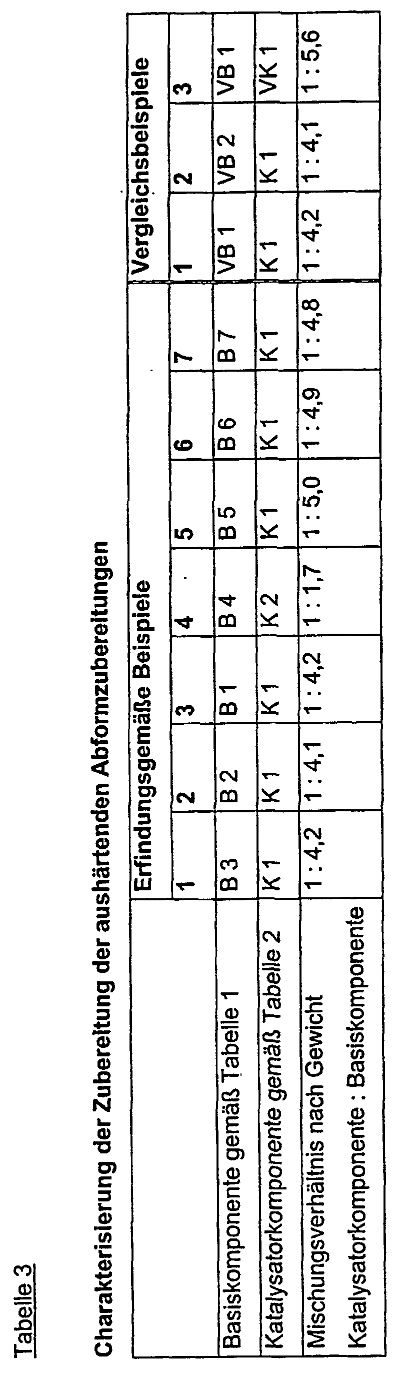

**Tabelle 4**

| Beurteilung der Entformbarkeit | | | |
|---|---|---|---|
| | | **Entnehmbarkeit aus dem Mund** (Durchschnittswerte) | **Entformbarkeit des Gips-Modells** (Durchschnittswerte) |
| Erfindungsbeispiel | | | |
| | 1 | 1,6 | 1,7 |
| | 2 | 1,3 | 1,3 |
| | 3 | 1,4 | 1,3 |
| | 4 | 1,5 | 1,7 |
| | 5 | 1,6 | 1,7 |
| | 6 | 1,4 | 1,7 |
| | 7 | 1,7 | 2,0 |

| Vergleichsbeispiel | | | |
|---|---|---|---|
| | 1 | 4,3 | 3,7 |
| | 2 | 4,8 | 4,2 |
| | 3 | 2,7 | 2,7 |

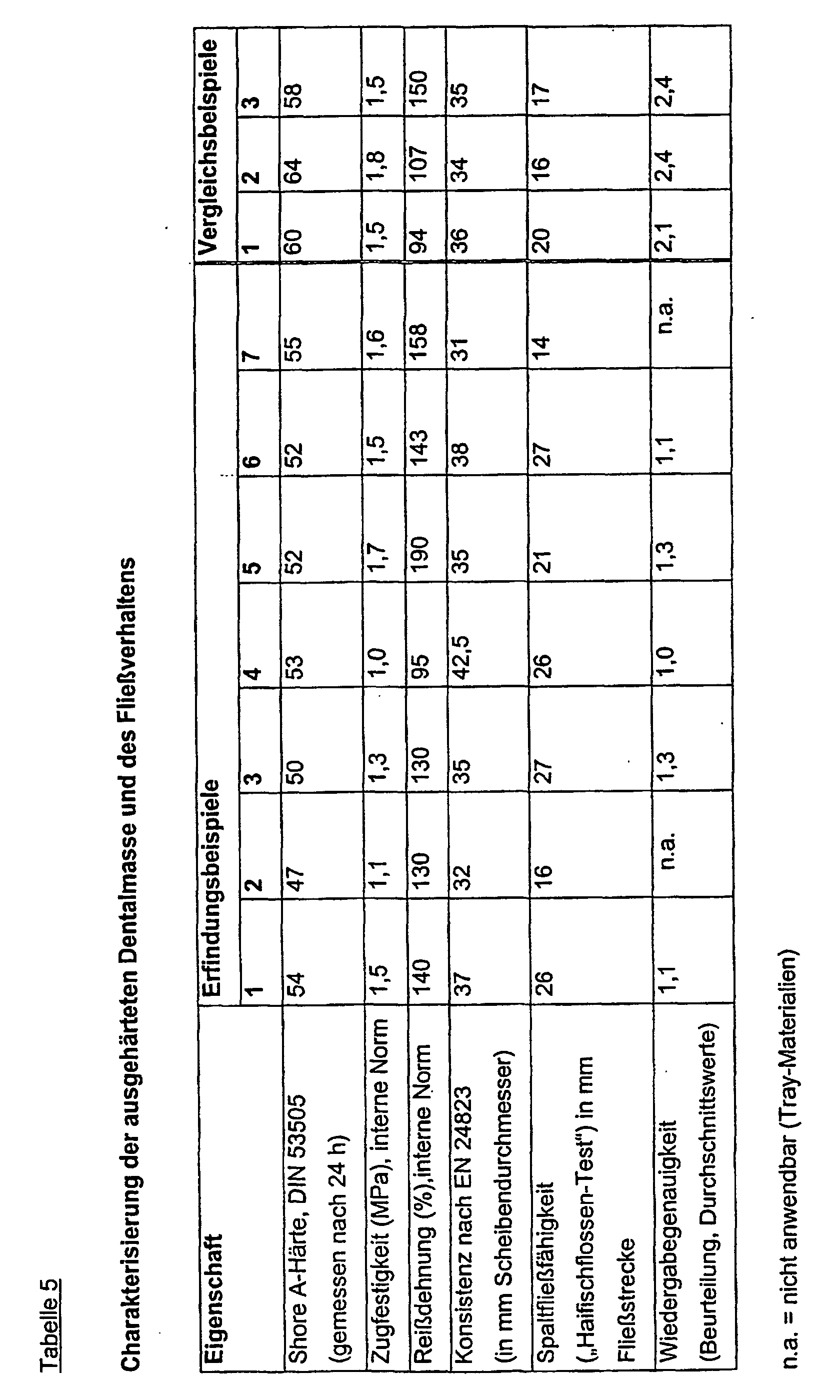

## Patentansprüche

1. Zubereitung, enthaltend
(A) 30 bis 56 Gew.-% Aziridinopolyether mit einem Gehalt von cyclischen Polyethem kleiner als 5,0 Gew.-%;
(B) 30 bis 45 Gew.-% Verbindungen, die eine Weichstellung der ausgehärteten Dentalmassen bewirken;
(C) 10 bis 15 Gew.-% Füllstoffe;
(D) 4 bis 10 Gew.-% weitere Wirkstoffe;
mit den Maßgaben, dass
• das Gewichtsverhältnis zwischen den Bestandteilen (A) und (C) zu den Verbindungen des Bestandteils (B) 1,2 bis 2,1 beträgt;
• der Bestandteil (B) aus Verbindungen mit Molmassen kleiner 500 g/Mol (B1) und aus Trisacylestern des Glycerins nicht tierischen Ursprungs mit Molmassen zwischen 500 bis 2000 g/Mol (B2) sowie aus Verbindungen mit Molmassen größer 2000 g/Mol (B3) besteht und
• das Gewichtsverhältnis zwischen (B1) und (B3) 1 : 0,8 bis 1 : 2,3 beträgt.

2. Zubereitung gemäß Anspruch 1, wobei als Bestandteil (A) Bis-Aziridinopolyether mit Aziridinoäquivalentmassen von 2000 bis 4000 g/Äquivalent verwendet werden, der Polyetherteil aus Oxytetramethylen- und Oxydimethylen-Einheiten im Verhältnis 4 : 1 bis 3 : 1 besteht und der Anteil an oligomeren cyclischen Ethern in den Bis-Aziridinopolyethern kleiner als 0,5 Gew.-% ist.

3. Zubereitung gemäß Anspruch 1, wobei der Bestandteil (B1) typische Weichmacher vom Estertyp, wie Ethyl- oder Butylester der Citronensäure oder der Acetylcitronensäure, Phthalsäureester längerer verzweigter Alkohole, Dialkylester von Dicarbonsäuren, wie Bis(2-ethylhexyl)-adipat, aromatische und aliphatische Sulfonsäureester, wie Alkylsulfonsäureester des Phenols oder von Alkanolen und typische aromatische Weichmacher, wie Polyphenyle, Dibenzyltoluol, Isomerengemische von C₂₀- bis C₃₀-Aromaten umfasst und hierbei die Verwendung von Gemischen aus Weichmachern des Estertyps und des aromatischen Typs bevorzugt ist.

4. Zubereitung gemäß Anspruch 1, wobei Trisacylester des Glycerins gemäß Bestandteil (B2) verwendet werden und hierbei künstliche Fette mit einem Steaorylgehalt von mehr als 65 Gew.-% des Triglycerids bevorzugt sind.

5. Zubereitung gemäß Anspruch 1, wobei als flüssige Polymere gemäß Bestandteil (B3) solche vom Polyethertyp, Polyestertyp, Polyurethantyp, Polycarbonattyp, Polyolefintyp eingesetzt werden und hierbei als Endgruppen Hydroxyl-, Ether-, Alkyl- und Acylgruppen bevorzugt sind.

6. Zubereitung gemäß Anspruch 5, wobei die flüssigen Polymeren vom Polyethertyp die gleiche oder eine ähnliche Zubereitung und Molmasse besitzen, wie die als Bestandteil (A) verwendeten Aziridinopolyether, jedoch Hydroxyl- oder Acetyl-Endgruppen tragen.

7. Zubereitung gemäß Anspruch 5, wobei die flüssigen Polymeren vom Polyethertyp Polypropylenoxidpolyole sind.

8. Zubereitung gemäß Anspruch 5, wobei die flüssigen Polymeren vom Polyethertyp statistische Copolymerisate und bzw. oder Blockcopolymerisate von Ethylenoxid und Propylenoxid mit Hydroxyl- oder Acetyl-Endgruppen sind.

9. Zubereitung gemäß einem der Ansprüche 1 bis 8, wobei als Bestandteil (C) Gemische von feinteiligen Kieselsäuren natürlichen und künstlichen Ursprungs verwendet werden und hierbei Diatomeenerde und pyrogene, oberflächenmodifizierte Kieselsäuren mit BET-Oberflächen von 100 bis 300 m²/g bevorzugt sind.

10. Verwendung von Zubereitungen gemäß einem der Ansprüche 1 bis 9 zur Herstellung von Dentalmassen.

11. Verwendung von Dentalmassen gemäß Anspruch 10 zur Abformung im zahnmedizinischen oder zahntechnischen Bereich.

12. Behältnis, enthaltend mindestens eine Dentalmasse, hergestellt aus Zubereitungen gemäß einem der Ansprüche 1 bis 9.

13. Mischvorrichtung, enthaltend mindestens eine Dentalmasse, hergestellt aus Zubereitungen gemäß einem der Ansprüche 1 bis 9.

## Claims

1. Preparation comprising
(A) 30 to 56 wt.-% aziridino polyethers with a cyclic polyether content lower than 5.0 wt.-%;
(B) 30 to 45 wt.-% compounds which effect a softening of the cured dental materials;
(C) 10 to 15 wt.-% fillers;
(D) 4 to 10 wt.-% further active ingredients;
with the provisos that
• the weight ratio between constituents (A) and (C) to the compounds of constituent (B) is 1.2 to 2.1;
• constituent (B) consists of compounds with molar masses lower than 500 g/mol (B1) and of trisacyl esters of glycerol of non-animal origin with molar masses between 500 to 2000 g/mol (B2) as well as of compounds with molar masses greater than 2000 g/mol (B3) and
• the weight ratio between (B1) and (B3) is 1:0.8 to 1:2.3.

2. Preparation according to claim 1, in which bis-aziridino polyethers with aziridino equivalent masses of 2000 to 4000 g/equivalent are used as constituent (A), the polyether part consists of oxytetramethylene and oxydimethylene units in the ratio 4:1 to 3:1 and the proportion of oligomeric cyclic ethers in the bis-aziridino polyethers is lower than 0.5 wt.-%.

3. Preparation according to claim 1, in which constituent (B1) comprises typical plasticizers of ester type, such as ethyl or butyl esters of citric acid or acetyl citric acid, phthalic acid esters of longer branched alcohols, dialkyl esters of dicarboxylic acids, such as bis(2-ethylhexyl)-adipate, aromatic and aliphatic sulphonic acid esters, such as alkylsulphonic acid esters of phenol or of alkanols and typical aromatic plasticizers, such as polyphenyls, dibenzyl-toluene, isomer mixtures of C₂₀ to C₃₀ aromatic compounds and the use of mixtures of plasticizers of the ester type and of the aromatic type is preferred.

4. Preparation according to claim 1, in which trisacyl esters of glycerol according to constituent (B2) are used and synthetic fats with a stearoyl content of more than 65 wt.-% of the triglyceride are preferred.

5. Preparation according to claim 1, in which liquid polymers according to constituent (B3) of polyether type, polyester type, polyurethane type, polycarbonate type, polyolefin type are used and hydroxyl, ether, alkyl and acyl groups are preferred as end groups.

6. Preparation according to claim 5, in which the liquid polymers of polyether type have the same or a similar preparation and molar mass as the aziridino polyethers used as constituent (A), but bear hydroxyl or acetyl end groups.

7. Preparation according to claim 5, in which the liquid polymers of the polyether type are polypropylene oxide polyols.

8. Preparation according to claim 5, in which the liquid polymers of the polyether type are random copolymers and/or block copolymers of ethylene oxide and propylene oxide with hydroxyl or acetyl end groups.

9. Preparation according to one of claims 1 to 8, in which mixtures of finely dispersed silicic acids of natural and synthetic origin are used as constituent (C) and diatomaceous earth and pyrogenic, surface-modified silicic acids with BET surfaces of 100 to 300 m²/g are preferred.

10. Use of preparations according to one of claims 1 to 9 for the preparation of dental materials.

11. Use of dental materials according to claim 10 for modelling in the field of dentistry or dental engineering.

12. Receptacle, containing at least one dental material, produced from preparations according to one of claims 1 to 9.

13. Mixing device, containing at least one dental material, produced from preparations according to one of claims 1 to 9.

## Revendications

1. Composition, contenant
(A) 30 à 56% en poids d'aziridinopolyéthers présentant une teneur en polyéthers cycliques inférieure à 5,0% en poids ;
(B) 30 à 45% en poids de composés qui provoquent un réglage de la souplesse des masses dentaires durcies ;
(C) 10 à 15% en poids de charges ;
(D) 4 à 10% en poids d'autres substances actives, ;
à condition que
- le rapport pondéral des constituants (A) et (C) aux composés du constituant (B) soit de 1,2 à 2,1,
- le constituant (B) soit constitué par des composés présentant des masses molaires inférieures à 500 g/mole (B1) et des esters de triacyle du glycérol qui ne sont pas d'origine animale, présentant des masses molaires entre 500 à 2000 g/mole (B2) ainsi que par des composés présentant des masses molaires supérieures à 2000 g/mole (B3) et
- le rapport pondéral entre (B1) et (B3) soit de 1:0,8 à 1:2,3.

2. Composition selon la revendication 1, dans laquelle on utilise comme constituant (A) des bis-aziridinopolyéthers avec des masses d'équivalent aziridino de 2000 à 4000 g/équivalent, la partie polyéther est constituée par des unités oxytétraméthylène et oxydiméthylène dans un rapport 4:1 à 3:1 et la proportion d'éthers cycliques oligomères dans les bis-aziridinopolyéthers est inférieure à 0,5% en poids.

3. Composition selon la revendication 1, dans laquelle le constituant (B1) comprend des plastifiants caractéristiques de type ester, tels que l'ester éthylique ou butylique de l'acide citrique ou de l'acide acétylcitrique, des esters de l'acide phtalique d'alcools ramifiés à longue chaîne, les esters dialkyliques d'acides dicarboxyliques, tels que l'adipate de bis(2-éthylhexyle), les esters d'acides sulfoniques aromatiques et aliphatiques, tels que les esters de l'acide alkylsulfonique du phénol ou des alcanols et des plastifiants aromatiques caractéristiques, tels que les polyphénols, le dibenzyltoluène, les mélanges d'isomères d'aromatiques en C₂₀ à C₃₀ et l'utilisation de mélanges de plastifiants du type ester et du type aromatique étant préférée ici.

4. Composition selon la revendication 1, dans laquelle on utilise des esters de triacyle du glycérol selon le constituant (B2) et en préférant les graisses synthétiques présentant une teneur en stéaroyle supérieure à 65% en poids du triglycéride.

5. Composition selon la revendication 1, dans laquelle on utilise comme polymères liquides selon le constituant (B3) ceux du type polyéther, polyester, polyuréthane, polycarbonate, polyoléfine, et en préférant ici comme groupes terminaux les groupes hydroxyle, éther, alkyle et acyle.

6. Composition selon la revendication 5, dans laquelle les polymères liquides du type polyéther présentent une composition et une masse molaire identiques ou analogues à celles des aziridinopolyéthers utilisés comme constituant (A), mais en portant des groupes terminaux hydroxyle ou acétyle.

7. Composition selon la revendication 5, dans laquelle les polymères liquides du type polyéther sont des poly(oxyde de propylène)-polyols.

8. Composition selon la revendication 5, dans laquelle les polymères liquides du type polyéther sont des copolymères statistiques et/ou des copolymères à blocs d'oxyde d'éthylène et d'oxyde de propylène avec des groupes terminaux hydroxyle ou acétyle.

9. Composition selon l'une quelconque des revendications 1 à 8, dans laquelle on utilise comme constituant (C) des mélanges de silices finement divisées d'origine naturelle et synthétique et en préférant les terres de diatomées et les silices pyrogènes, à surface modifiée présentant des surfaces BET de 100 à 300 m²/g.

10. Utilisation de compositions selon l'une quelconque des revendications 1 à 9 pour la préparation de masses dentaires.

11. Utilisation de masses dentaires selon la revendication 10 pour le moulage dans le domaine de la médecine dentaire ou de la technique dentaire.

12. Conteneur contenant au moins une masse dentaire préparée à partir des compositions selon l'une quelconque des revendications 1 à 9.

13. Dispositif de mélange contenant au moins une masse dentaire préparée à partir des compositions selon l'une quelconque des revendications 1 à 9.
